# EUROPÄISCHE PATENTSCHRIFT

(11) **EP 2 640 504 B1**
(45) Veröffentlichungstag und Bekanntmachung des Hinweises auf die Patenterteilung: **09.05.2018**
(21) Anmeldenummer: 11785384.6
(22) Anmeldetag: 15.11.2011
(51) Int. Cl.: B01J 8/02, B01J 8/44

(54) **CHEMISCHER REAKTOR MIT DRAHTGESTRICK-MASCHENWARE ALS HALTEEINRICHTUNG FÜR PARTIKEL**
CHEMICAL REACTOR WITH KNITTED WIRE MESH FABRIC AS A HOLDING DEVICE FOR PARTICLES
RÉACTEUR CHIMIQUE COMPRENANT UNE MATIÈRE À MAILLES EN FIL TRICOTÉ COMME DISPOSITIF DE RETENUE POUR PARTICULES

(30) Priorität: 18.11.2010 DE 102010044111
(43) Veröffentlichungstag der Anmeldung: 25.09.2013
(73) Patentinhaber: Covestro Deutschland AG, 51373 Leverkusen (DE)
(72) Erfinder: MERKEL, Michael, 40223 Düsseldorf (DE); WILKE, Karl-Heinz, 47447 Moers (DE); KNAUF, Thomas, 41542 Dormagen (DE)
(74) Vertreter: Levpat
(86) Internationale Anmeldenummer: PCT/EP2011/070101
(87) Internationale Veröffentlichungsnummer: WO 2012/065969

(56) Entgegenhaltungen:
- EP-A1- 2 327 473
- DE-A1- 19 706 434

## Beschreibung

Die vorliegende Erfindung betrifft einen chemischen Reaktor zur heterogen katalysierten Umsetzung eines Fluids mit einer verbesserten Halteeinrichtung für vom Fluid durchströmte inerte Partikel und/oder Katalysatorpartikel. Sie betrifft weiterhin ein Verfahren zur Durchführung von chemischen Reaktionen mittels eines solchen Reaktors und die Verwendung von Drahtgestrick-Maschenware in chemischen Reaktoren.

Die Hydrierung von Nitroaromaten zu den entsprechenden Aminen in einem axial durchströmten Festbettreaktor kann in einem mehrstufigen Prozess unter adiabatischen Reaktionsbedingungen durchgeführt werden. Solche Hydrierungen verlaufen in periodischen Zyklen, wobei sich Produktionszyklen mit Regenerationszyklen abwechseln, in denen zum Beispiel kohlenstoffhaltige Ablagerungen durch Abbrennen entfernt werden.

Reaktoren für solche Reaktionen haben typischerweise eine zylindrische Form und sind mit einem Spaltsieb ausgestattet, dass als Unterlage für den Festbettkatalysator dient (Heterogeneous Catalysis in Industrial Practice, 2nd Ed. 1996, Seite 474). Diese Spaltsiebe müssen das Gewicht des Katalysators sowie die zusätzliche Belastung, die sich durch den Druckverlust über die Katalysatorschüttung ergibt, tragen.

Häufige Verwendung finden dabei Spaltsiebe, wie sie zum Beispiel in US 2,915,375 beschrieben werden. Sie bestehen aus parallel angeordneten V-förmigen Drähten, die ihrerseits wieder unterstützt und fixiert werden, so dass sich Spalte ergeben, deren Weite kleiner ist als die Korngröße des zu stützenden Materials und die sich nach unten hin verbreitern, um gegebenenfalls zwischen die Drähte gelangendes Material nach unten hin abzuführen und eine Verstopfung des Spaltsiebes zu vermeiden.

Durch den periodischen Betrieb der Reaktoren ist das Sieb thermischen Spannungen unterworfen, die zu Beschädigungen führen. Dies ist insbesondere dann der Fall, wenn exotherme Reaktionen unter adiabaten Bedingungen durchgeführt werden, also die freiwerdende Reaktionswärme vom Reaktionsgas aufgenommen wird, so dass es zu großen Temperatursprüngen im inneren des Reaktors kommt.

In diesen Fällen kann es nach wenigen Produktionszyklen schon zu Verformungen der V-förmigen, ursprünglich parallel angeordneten Drähte, bis hin zum Ab- oder Zerreißen einzelner Drähte kommen. Die Reparatur eines so beschädigten Siebes ist aufwändig und bringt keinen dauerhaften Erfolg. Um diese Problematik zu minimieren, werden die Spaltsiebe in der Regel an einem schwimmend gelagerten Stützring befestigt, der einen Spalt zur Reaktorwand hin aufweist, um thermische Ausdehnungen auszugleichen.

Gerade bei größeren Reaktoren ist eine absolut ebene Auflagefläche (Tragring) für diesen Ring, wenn überhaupt, nur mit erheblichem Aufwand zu fertigen. Zudem ist es schwierig große Reaktoren exakt rund herzustellen. Als Folge dieser Probleme tritt häufig ein Katalysatorverlust in der Form auf, dass der Katalysator durch Beschädigungen des Spaltsiebs oder durch Öffnungen zwischen Spaltsieb und Reaktorwand beziehungsweise Tragring rieselt.

Verschärft wird die Problematik noch durch den Einsatz immer kleinerer Katalysatorpartikel, die im Allgemeinen eine höhere Effizienz aufweisen, aber auch leichter durch kleine Fehlstellen rieseln. An der Oberfläche der Schüttung entstehen durch den Katalysatorverlust thrombenförmige Vertiefungen. Das Reaktionsgas strömt dann bevorzugt durch die Bereiche mit den Vertiefungen, so dass eine ungleichmäßige Gasströmung entsteht, bis hin zu einem Umgang (Bypass) nicht umgesetzten Eduktes. Des Weiteren kann der auf die Rückseite des Spaltsiebs gewanderte Katalysator Beschädigungen an dahinter befindlichen Apparaten hervorrufen.

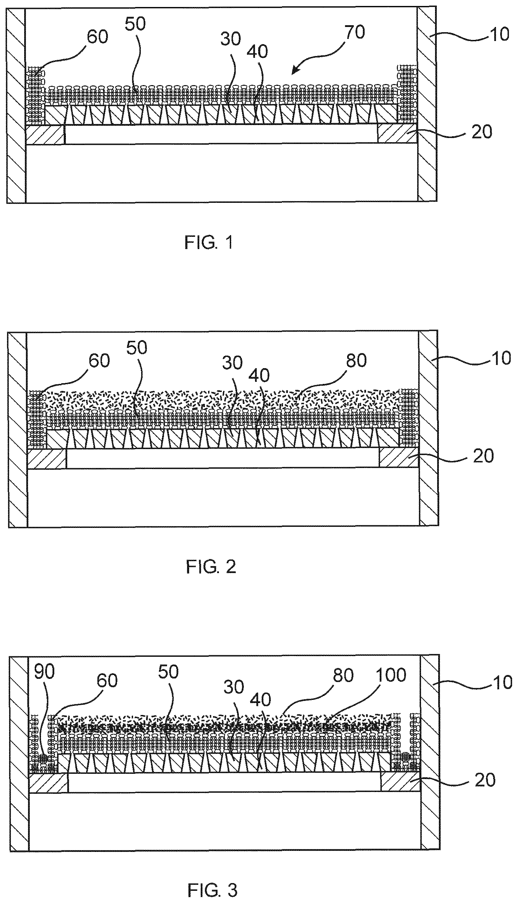

Um einen Katalysatordurchtritt auf die andere Seite des Spaltsiebes zu vermeiden, kann der Katalysator auf einer komplexen, mehrschichtigen Schüttung aus Inertpartikeln gelagert werden, wobei der Partikeldurchmesser in der Schicht, die dem Katalysator am nächsten liegt, etwas größer ist als der Partikeldurchmesser des Katalysators und dann mit jeder Schicht zunimmt (Fixed-Bed Reactor Design and Diagnostics, 1990, Fig. 1.1, Seite 4).

Auf diese Weise wird erreicht, dass auf dem Spaltsieb zunächst recht große Inertpartikel liegen, die bei kleineren Fehlstellen noch nicht durch das Sieb rieseln können. Nachteilig an dieser Lösung ist einerseits, dass ein nicht unerheblicher Teil des Reaktors mit Inertmaterial gefüllt wird und nicht mehr für den eigentlichen Katalysator zur Verfügung steht und andererseits, dass das Einbringen der verschiedenen Schichten in den Reaktor aufwändig ist. Soll das Inertmaterial bei einer Neubefüllung des Reaktors wiederverwendet werden, so erfordert dies ein mehrstufiges Sieben des aus dem Reaktor entnommenen Materials, um die unterschiedlich großen Partikel voneinander zu trennen.

Es war daher Aufgabe der Erfindung, einen Reaktor bereitzustellen, bei dem auch im Falle wiederholter thermischer Beanspruchung ein Durchtritt von Katalysator auf die Rückseite der Tragkonstruktion für den Katalysator verhindert wird, wobei die Tragkonstruktion kostengünstig, der Instandhaltungsaufwand gering und das Befüllen des Reaktors mit der Katalysatorschüttung einfach sein soll.

Erfindungsgemäß wird die Aufgabe gelöst durch einen chemischen Reaktor zur heterogen katalysierten Umsetzung eines Fluids, umfassend eine vom Fluid durchströmte Halteeinrichtung für Partikel, wobei die in Strömungsrichtung des Fluids gesehen stromaufwärts gelegene Seite der Halteeinrichtung Drahtgestrick-Maschenware umfasst, auf der Katalysatorpartikel angeordnet sind oder auf der eine Schicht von gegenüber dem Fluid inerten Partikeln angeordnet ist und auf dieser Schicht Katalysatorpartikel angeordnet sind, wobei die Drahtgestrick-Maschenware auf einem Träger angeordnet ist und der Träger in Strömungsrichtung des Fluids durchgehende Öffnungen umfasst, und wobei die durchschnittliche lichte Maschenweite des Drahtgestricks kleiner als die mittlere Partikelgröße X_{50.3} der Partikel ist, welche durch eine mit einer Vibrationssiebmaschine mit Siebsatz durchgeführte Siebanalyse unter Einhaltung der Bestimmungen der DIN 66165 (Fassung vom April 1987) ermittelt wird.
*Maschenwaren* sind beispielsweise aus der Textil- und Schmuckindustrie bekannt. Ganz allgemein bezeichnet eine Maschenware ein Flächengebilde, bei denen eine mittels eines Fadens oder mehrerer Fäden gebildete Schleife in eine andere Schleife hineingeschlungen ist, wobei die "Maschen" entstehen. Davon zu unterscheiden sind Gewebe, bei denen die Fläche durch Verkreuzen von zwei Fadensystemen hergestellt wird. Maschenware weist eine höhere Dehnbarkeit und Elastizität als Gewebe auf. Maschenwaren können in Strick-, Wirk- und Kulierwaren unterteilt werden. Für die Durchführung der vorliegenden Erfindung sind grundsätzlich alle drei geeignet. Besonders bevorzugt werden jedoch Strick- und/oder Kulierwaren eingesetzt, da diese noch elastischer sind als die (ohnehin schon elastischen) Wirkwaren. In der vorliegenden Erfindung kommt eine *Drahtgestrick-Maschenware* zum Einsatz, d. h. die erfindungsgemäße Maschenware wird aus Drähten und/oder Metallfäden gefertig. Dabei umfasst der Begriff "Draht*gestrick*" im Rahmen der vorliegenden Erfindung alle Arten von Maschenware, also auch Wirkware. Die Herstellung des Drahtgestricks erfolgt mit an sich bekannten Drahtverarbeitungsmaschinen, wie sie beispielsweise für Anwendungen in der Automobilindustrie, der Verfahrens- und Umwelttechnik gebräuchlich sind (z. B. Drahtstrickmaschinen). Geeignet für die Durchführung der vorliegenden Erfindung sind beispielsweise Drahtgestricke der Firma DHD Technology.
Durch die Flexibilität der Maschenware ist dafür gesorgt, dass sie im periodischen Betrieb bei unterschiedlichen Temperaturen nicht beschädigt wird. Gleichzeitig verhindert die 3-dimensionale Struktur der Maschenware, dass sich zu viele Maschen mit Partikeln zusetzen und der Gasfluss behindert wird.
Unter *Partikeln* können im Sinne dieser Erfindung sowohl *Katalysatorpartikel* (z. B. ein auf Aluminiumoxidkugeln geträgerter Edelmetallkatalysator) als auch *inerte Partikel* (z. B. Aluminiumoxidkugeln) verstanden werden. Bevorzugt werden Partikel eingesetzt, die mindestens im Wesentlichen kugelförmig sind, d. h., deren evtl. vorhandene Abweichungen von der idealen Kugelgeometrie so gering sind, dass sie sich in der Praxis wie ideale Kugeln verhalten. Grundsätzlich ist die Erfindung jedoch auch auf asymmetrisch geformte Partikel anwendbar. Als erster Anhaltspunkt für die mittlere Partikelgröße dienen die Angaben des Herstellers.

Für die genaue Bestimmung der *mittleren Partikelgröße* ist eine "Partikelgrößenanalyse" erforderlich. Im Rahmen dieser Erfindung erfolgt diese durch Siebanalyse. Erfindungsgemäß wird dabei als mittlere Partikelgröße der massebezogene Wert ("x_{50,3}") verwendet.

Zur Bestimmung der mittleren Partikelgröße im Rahmen der vorliegenden Erfindung wird nun so vorgegangen, dass man zunächst eine repräsentative Probe der zu stützenden Partikel einer Siebanalyse unterwirft und das Ergebnis massebezogen auswertet. Die Siebanalyse erfolgt dabei unter Verwendung einer Vibrationssiebmaschine (z. B. Modell AS 200 digit der Firma Retsch), in welcher die Analysensiebe mit aufsteigender Maschenweite übereinander zu einem Siebsatz angeordnet werden. Die Auswahl der Analysensiebe (Durchmesser und Maschenweite) hängt in erster Linie von der Siebgutmenge und der zu erwartenden (ggf. sind Vorversuche nötig) Partikelgrößenverteilung ab. Die Anzahl der Siebe und die Abstufungen der nominalen Öffnungsweiten sollten so ausgewählt werden, dass möglichst das gesamte Partikelspektrum der Probe in Fraktionen aufgeteilt wird. Bei der Durchführung der Siebanalyse ist sicherzustellen, dass der maximale Durchgang an Siebgut (optimaler Aussiebegütegrad) erzielt wird. Erforderlichenfalls (wenn beispielsweise neue Partikel, mit denen noch keine Betriebserfahrung vorliegt, eingesetzt werden sollen) müssen geeignete Siebzeiten und Amplituden in Vorversuchen experimentell ermittelt werden. Ein erster Anhaltspunkt für die Amplitude ergibt sich aus der Beobachtung der Siebgutbewegung. Diese sollte weder zu schwach noch zu stark sein. Die optimale Siebzeit ist erreicht, wenn sich die Masse des Siebdurchgangs in einer Minute um weniger als 0,1 % der Aufgabemenge verändert (DIN 66165, Fassung vom April 1987). Der Fachmann ist mit den hier nur kurz umrissenen Methoden vertraut.

Die Siebanalyse liefert als Ergebnis die Partikelgrößenverteilung der gemessenen Partikel. Das Ergebnis wird vorzugsweise graphisch dargestellt, indem der Massenanteil der einzelnen Fraktionen ("p₃") in einem Balkendiagramm und die aus den prozentualen Anteilen kumulierte Summenkurve ("Q₃") über den nominalen Sieböffhungsweiten (x) aufgetragen werden. Der Fachmann kann die mittlere Partikelgröße x_{50,3} (d. h. 50 Massen-% der Partikel sind kleiner als der entsprechende Wert x) leicht errechnen, entweder manuell oder bevorzugt durch Computer-gestützte Auswerteprogramme.

Zeigt dieses erste Resultat eine ausreichende Homogenität der Probe an, so ist die so ermittelte mittlere Partikelgröße x_{50,3} der zur Durchführung der Erfindung erforderliche Wert. Unter einer *ausreichenden Homogenität* wird in diesem Zusammenhang verstanden, dass die Partikelgrößenverteilung monomodal ist und dass jeweils maximal 0,1 Massen-% aller Partikel im Bereich zwischen ½ x_{50,3} und ²/₃ x_{50,3} bzw. im Bereich zwischen 1⅓ x_{50,3} und 1½ x_{50,3} liegen. Ferner sollten keine Partikel kleiner als ½ x_{50,3} oder größer als 1½ x_{50,3} sein. Zeigt dieses erste Resultat keine ausreichende Homogenität der Probe in obigem Sinne an, so werden die zu stützenden Partikel durch Siebung in großtechnischem Maßstab homogenisiert, d. h. Staubanteile und nicht mehr einsetzbare Bruchstücke werden abgetrennt. Dies wird so lange durchgeführt, bis die genannten Anforderungen an die Homogenität erfüllt sind.

Der chemische Reaktor kann beispielsweise ein axial durchströmter Festbettreaktor sein, wie er in der Hydrierung von Nitrobenzol zu Anilin eingesetzt wird. Das im Reaktor umzusetzende Fluid kann flüssig, gasförmig oder überkritisch sein. Es kann einen Reaktanden oder eine Mischung aus mehreren Reaktanden enthalten.

Wie später eingehender beschrieben werden wird, können die von der Drahtgestrick-Maschenware zurückgehaltenen Partikel sowohl Katalysatorpartikel als auch inerte Partikel sein.

Die Art der Drahtgestrick-Maschenware ist zunächst nicht weiter festgelegt. Es kann sich um einadrigen oder mehradrigen Draht handeln, der auf beliebige Weise verstrickt wurde. So können die Gestricke beispielsweise bis zu 5 runde, flache, glatte und/oder gewellte Fäden enthalten. Verstrickte Materialien können unter anderem Stahl, Edelstähle wie 1.4571, Kupfer, Monel (2.4360) oder Aluminium sein. Neben Drahtgestricken aus Flachstrickmaschinen können zum Beispiel auch zweilagige Drahtgestrickbahnen verwendet werden, die durch das Zusammendrücken von Drahtgestrickschläuchen, die mit Rundstrickmaschinen gefertigt werden, bezüglich einer durch die Mittelachse der Drahtgestrickschläuche verlaufenden Faltebene entstehen.

Erfindungsgemäß ist vorgesehen, dass die durchschnittliche lichte Maschenweite des Drahtgestricks kleiner ist als die mittlere Partikelgröße x_{50,3} der Partikel, welche in der Halteeinrichtung zur Aufnahme bestimmt sind. Sie bezeichnet den Innenabstand (nicht den Abstand von Drahtmitte zu Drahtmitte) zweier benachbarter Maschenschenkel am Punkt der größten Ausdehnung einer Masche. Bevorzugt liegt das Verhältnis zwischen Maschenlänge, also dem Abstand zweier benachbarter Maschenköpfe, und Maschenweite zwischen 4 : 1 und 0,5 : 1, besonders bevorzugt zwischen 2 : 1 und 1 : 1. Die durchschnittliche lichte Maschenweite bezeichnet dann den Durchschnittswert aller lichten Weiten einzelner Maschen einer Drahtgestrick-Maschenware. Die durchschnittliche lichte Maschenweite wird bei der Herstellung der Drahtgestrick-Maschenware nach dem Fachmann bekannten Methoden eingestellt (z. B. Anzahl, Abstand und Dicke der Nadeln am Strickkopf, Anzahl und Stärke der Drähte usw.). Die Verwendung von Maschenware mit uneinheitlicher Maschwenweite (d. h. Unterschieden in den Maschenweiten, die über normale fertigungsbedingte Toleranzen hinausgehen) ist nicht bevorzugt, aber grundsätzlich auch möglich. In diesem Fall ist bevorzugt die lichte Maschenweite der größten Masche kleiner als die mittlere Partikelgröße x_{50,3}.

Die Halteeinrichtung für Partikel kann im einfachsten Fall durch im Reaktor aufgespannte Drahtgestrick-Maschenware alleine gebildet werden. Es ist jedoch bevorzugt, dass die Drahtgestrick-Maschenware durch einen Träger unterstützt wird, um den auftretenden mechanischen Belastungen gewachsen zu sein. Dann sind sowohl der Träger als auch die Drahtgestrick-Maschenware Teil der Halteeinrichtung für Partikel. Zusammen mit Katalysatorpartikeln wird hieraus das Katalysatorbett gebildet.

Die Lage der Drahtgestrick-Maschenware in der Halteeinrichtung für Partikel im vom Fluid durchströmten Reaktor ergibt sich aus der Funktion der Maschenware zur Zurückhaltung von Partikeln. Sie befindet sich daher auf der stromaufwärts gelegenen Seite der Halteeinrichtung.

Vorteilhaft bei dem erfindungsgemäßen Reaktor ist weiterhin, dass ein einfaches Spaltsieb als Träger verwendet werden kann. Die Verwendung eines aufwändig gefertigten Spaltsiebes ist nicht erforderlich, sondern es kann auch ebenfalls ein einfacher Rost zur Anwendung kommen. Die Maschenware wird auf das Spaltsieb oder den Rost aufgelegt und gegebenenfalls fixiert.

Die aufgelegte Maschenware übernimmt nun die Funktion der Katalysatorrückhaltung. Dadurch kann nicht nur eine Beschädigung des Spaltsiebes in Kauf genommen werden, sondern die Öffnungen im Spaltsieb können von vornherein größer ausgelegt werden.

Hieraus ergibt sich ein weiterer Vorteil: Durch die größeren Öffnungen ist es möglich, die Dicke des Spaltsiebes oder Rostes zu erhöhen, ohne dass Verstopfungen zu befürchten sind. Dadurch wird wiederum die Steifigkeit der die Maschenware tragenden Konstruktion erhöht, so dass höhere Katalysatormengen getragen werden können und auch höhere Druckverluste und die sich daraus ergebenden Lasten verkraftet werden. Es kann sogar auf die Schüttung mit Inertmaterial verzichtet werden, wodurch nochmals höhere Katalysatormengen zum Einsatz kommen können.

Ausführungsformen der Erfindung werden nachfolgend geschildert, wobei die Ausführungsformen frei miteinander kombiniert werden können, sofern sich nicht aus dem Zusammenhang eindeutig das Gegenteil ergibt.

In einer Ausführungsform des erfindungsgemäßen Reaktors beträgt die durchschnittliche lichte Maschenweite des Drahtgestricks ≥ 20 % bis ≤ 80 % der mittleren Partikelgröße x_{50,3}. Vorzugsweise beträgt dieser Wert ≥ 30 % bis ≤ 60 % und mehr bevorzugt ≥ 40 % bis ≤ 50 %. Auf diese Weise wird besonders verhindert, dass durch in den Maschen verfangene Partikel ein unerwünschter Druckverlust während des Betriebes des Reaktors auftritt.
In einer weiteren Ausführungsform des erfindungsgemäßen Reaktors beträgt die Dehnbarkeit der Drahtgestrick-Maschenware unter mechanischer und/oder thermischer Belastung in Flächenrichtung ≥ 1 %, bevorzugt ≥ 2 %. Die Dehnbarkeit in Flächenrichtung sollte 50 %, bevorzugt 25 %, bevorzugt 10 % nicht überschreiten. Auf diese Weise wird besonders sichergestellt, dass sich die Maschenware den thermischen Ausdehnungen des Reaktors anpasst ohne Schaden zu nehmen und ohne, dass Partikel durch die Maschen fallen.
In einer weiteren Ausführungsform des erfindungsgemäßen Reaktors beträgt der Drahtdurchmesser der Drahtgestrick-Maschenware ≥ 0,03 mm bis ≤ 1 mm. Vorzugsweise beträgt der Durchmesser des Drahts, aus dem die Maschenware gestrickt ist, ≥ 0,1 mm bis ≤ 0,5 mm und mehr bevorzugt ≥ 0,2 mm bis ≤ 0,3 mm. Auf diese Weise wird besonders sichergestellt, dass der Draht eine ausreichende Stabilität bei gleichzeitig geringem Gewicht und großer Durchgangsfläche für das Fluid aufweist.
In einer weiteren Ausführungsform des erfindungsgemäßen Reaktors beträgt die Höhe der Drahtgestrick-Maschenware in der Halteeinrichtung für Partikel ≥ 3 mm bis ≤ 100 mm. Dieses lässt sich beispielsweise durch mehrfach gefaltetes Auflegen der Maschenware auf einem Träger erreichen. Vorzugsweise beträgt die Höhe ≥ 5 mm bis ≤ 80 mm und mehr bevorzugt ≥ 10 mm bis ≤ 50 mm. So wird der Durchtritt von Teilchen auf die stromabwärts liegende Seite der Maschenware besonders zuverlässig verhindert und es verbleiben stets freie Kanäle für das durchströmende Fluid.
In einer weiteren Ausführungsform des erfindungsgemäßen Reaktors ist die Drahtgestrick-Maschenware eine durch mechanische Einwirkung verformte Drahtgestrick-Maschenware. Hierzu kann die Maschenware gewalzt oder zwischen zwei Zylinderrollen oder Zahnrädern hindurchgeführt werden. Auf diese Weise verformte Maschenware hat entweder eine geringe Höhe, um den verfügbaren Raum für Katalysator im Reaktor zu maximieren (gewalzt) oder erhält eine stärker dreidimensionale Struktur (durch Zahnräder), so dass mit geringem Materialaufwand eine höhere Auflage aus Maschenware erreicht werden kann. Diese weist dann einen besonders geringen Druckverlust und eine besonders geringe Neigung zur Verstopfung der Maschen auf. Im erfindungsgemäßen Reaktor ist die Drahtgestrick-Maschenware auf einem Träger angeordnet und der Träger umfasst in Strömungsrichtung des Fluids durchgehende Öffnungen. Wie bereits geschildert sind bevorzugte Träger Spaltsiebe und Gitterroste. Der Träger lässt sich auf einem Tragring auf der Innenseite der Reaktorwand schwimmend lagern, um die thermische Ausdehnung während des Betriebes auszugleichen.
Vorzugsweise ist hierbei zwischen dem Träger und der Wand des Reaktors zusätzlich Drahtgestrick-Maschenware angeordnet. Hierdurch wird ein zwecks Kompensation der thermischen Ausdehnung bestehender Spalt gegen Partikeldurchtritt gesichert. Vorzugsweise ist diese Drahtgestrick-Maschenware eine metallische Dichtschnur aus Maschenware. Es ist günstig, wenn sie aus demselben Material wie die auf dem Träger befindliche Maschenware besteht.
In einer besonders bevorzugten Ausführungsform des erfindungsgemäßen Reaktors ist die zwischen dem Träger und der Wand des Reaktors zusätzlich befindliche Drahtgestrick-Maschenware U-förmig angeordnet und eine elastisch vorgespannte weitere Drahtgestrick-Maschenware ist in die hierdurch gebildete Vertiefung eingebracht. Beispielsweise kann dieses eine metallische Dichtschnur aus Maschenware sein.
Die Schenkel des "U" können jeder für sich oder zusammen senkrecht stehen, um beispielsweise der Form der Reaktorwand angepasst zu sein. Es ist aber auch möglich, dass die Schenkel zumindest teilweise einen waagerechten Verlauf nehmen. Dann können sie auf einem Spaltsieb oder Gitterrost aufliegen. Hierauf kann dann weitere Maschenware gelegt werden.
Die Art der Vorspannung kann sowohl eine Kompression als auch eine Dehnung sein. Vorzugsweise wird die Dichtschnur vor dem Einbringen komprimiert, so dass sie sich anschließend ausdehnt. Dann werden die beiden nach oben stehenden Seiten des "U" an den Träger und/oder einen Tragring sowie an die Reaktorwand angedrückt. Damit wird sicher und dauerhaft vermieden, dass Inert- oder Katalysatorpartikel etwaige Fehlstellen zwischen Träger, Tragring und Reaktorwand beziehungsweise Auflagefläche des Tragringes passieren. Die metallische Dichtschnur ist dabei federelastisch und gleicht zum einen die Passungenauigkeit zwischen Spaltsieb und Reaktorwand und zum anderen die Änderungen in der Breite des Spaltes zwischen Spaltsieb und Reaktorwand auch bei Temperaturwechseln aus.

Im erfindungsgemäßen Reaktor sind in der Halteeinrichtung - in einer Alternative der Erfindung - Katalysatorpartikel auf der Drahtgestrick-Maschenware angeordnet. Geeignete Katalysatoren sind unter anderem in EP 0 011 090 A1 beschrieben und umfassen:
(a)1 - 100 g/l Träger mindestens eines Metalls der Gruppen 8 bis 12 des Periodensystems der Elemente, und
(b)0 - 100 g/l Träger mindestens eines Übergangsmetalls der Gruppen 4 bis 6 und 12 des Periodensystems der Elemente, sowie
(c)0 - 100 g/l Träger mindestens eines Metalls der Hauptgruppenelemente der Gruppen 14 und 15 des Periodensystems der Elemente
auf einem Aluminiumoxid-Träger mit einer BET-Oberfläche von weniger als 20 m²/g.
Im erfindungsgemäßen Reaktor ist in der Halteeinrichtung - in einer weiteren Alternative der Erfindung - auf
der Drahtgestrick-Maschenware eine Schicht von gegenüber dem Fluid inerten Partikeln angeordnet und es sind auf dieser Schicht Katalysatorpartikel angeordnet. Gegenüber dem Fluid inerte Partikel sind katalytisch inaktiv und reagieren nicht mit dem Fluid. Wenn solche Partikel als Zwischenschicht auf der Maschenware aufliegen, ist es möglich, kleinere und somit aktivere Katalysatorpartikel einzusetzen.

Ein weiterer Gegenstand der vorliegenden Erfindung ist ein Verfahren zur Umsetzung eines Fluids, wobei die Umsetzung in einem erfindungsgemäßen Reaktor in Gegenwart von heterogenen Katalysatorpartikeln durchgeführt wird und die Katalysatorpartikel in der Halteeinrichtung für Katalysatorpartikel angeordnet sind. Mit eingeschlossen ist der Fall, dass sich eine Zwischenschicht aus inerten Partikeln im Katalysatorbett befindet. Der Vorteil des erfindungsgemäßen Verfahrens liegt insbesondere in längeren Standzeiten, bis eine erneute Wartung notwendig ist.

In einer Ausführungsform des erfindungsgemäßen Verfahrens wird weiterhin nach Beendigung der Umsetzung der Reaktor in Gegenwart von Sauerstoff auf eine Temperatur von ≥ 200 °C bis ≤ 400 °C erhitzt. Es hat sich gezeigt, dass erfindungsgemäße Reaktoren auch diesem Abbrennen von Kohlenstoffablagerungen in vorteilhafter Weise gewachsen sind.

In einer weiteren Ausführungsform des erfindungsgemäßen Verfahrens umfasst das Fluid aromatische Nitroverbindungen und Wasserstoff. Insbesondere kann es sich um die Hydrierung von Nitrobenzol zu Anilin handeln. Das erfindungsgemäße Verfahren kann länger betrieben werden, bis es zu einem erhöhten Nitrobenzolgehalt (Nitrobenzoldurchbruch) im Endprodukt kommt.

In einer weiteren Ausführungsform des erfindungsgemäßen Verfahrens wird die Umsetzung adiabatisch durchgeführt. Dieses Verfahren wird zum Beispiel in EP 0 696 573 A1 und EP 0 696 574 A1 beschrieben. Das erfindungsgemäße Verfahren ist den bei adiabaten Umsetzungen auftretenden Temperatursprüngen in vorteilhafter Weise gewachsen.

Ein zusätzlicher Aspekt der vorliegenden Erfindung ist die Verwendung von Drahtgestrick-Maschenware als Halteeinrichtung für Katalysatorpartikel und/oder inerte Partikel in chemischen Reaktoren, wie im Anspruch 12 spezifiziert. Zu Details und bevorzugten Ausführungsformen wird zur Vermeidung von Wiederholungen auf die Erläuterungen zum erfindungsgemäßen Reaktor verwiesen.
Die Erfindung wird anhand der nachfolgenden Zeichnungen und Beispiele weiter erläutert, ohne jedoch darauf beschränkt zu sein. Es zeigen:
- FIG. 1: einen erfindungsgemäßen chemischen Reaktor
- FIG. 2: einen weiteren erfindungsgemäßen chemischen Reaktor
- FIG. 3: einen weiteren erfindungsgemäßen chemischen Reaktor
- FIG. 4: einen weiteren erfindungsgemäßen chemischen Reaktor
- FIG. 5: einen weiteren erfindungsgemäßen chemischen Reaktor
- FIG. 6: einen weiteren erfindungsgemäßen chemischen Reaktor
- FIG. 7: ein Detail des in FIG. 6 gezeigten Reaktors
FIG. 1 zeigt schematisch eine Querschnittsansicht eines erfindungsgemäßen chemischen Reaktors, wie er beispielsweise als axial durchströmter Festbettreaktor für die Hydrierung von Nitrobenzol zu Anilin eingesetzt werden kann. Die Strömungsrichtung eines Reaktionsfluids verläuft in der Zeichnung vertikal von oben nach unten.
Auf der Innenseite der Reaktorwände 10 ist umlaufend ein Tragring 20 ausgebildet. Auf diesem Tragring 20 befindet sich schwimmend gelagert ein als Spaltsieb ausgebildeter Träger 30. Die Spalten 40 dieses Spaltsiebs weiten sich in Strömungsrichtung gesehen auf. Das Spaltsieb kann durch einen umlaufenden Stützring begrenzt sein.
Drahtgestrick-Maschenware 50 befindet sich auf dem Träger 30 und in dem Spalt, der sich zwischen dem Träger 30 und der Reaktorwand 10 befindet, ist zusätzliche Drahtgestrick-Maschenware 60 eingebracht.
Die durch den Träger 30 und die Drahtgestrick-Maschenware 50, 60 gebildete Halteeinrichtung kann in dem Bereich 70 Katalysatorpartikel aufnehmen. Die Drahtgestrick-Maschenware 50 befindet sich hierbei auf der in Strömungsrichtung eines Reaktionsfluids gesehen stromaufwärts gelegenen Seite der Halteeinrichtung.

FIG. 2 zeigt dieselbe Querschnittsansicht eines chemischen Reaktors wie FIG. 1 mit dem Unterschied, dass nunmehr Katalysatorpartikel 80 auf der Drahtgestrick-Maschenware 50 angeordnet sind. Die durchschnittliche lichte Maschenweite der Drahtgestrick-Maschenware 50 ist hierbei so bemessen, dass die Katalysatorpartikel 80 zurückgehalten werden.

FIG. 3 zeigt weitere Ausgestaltungen des in FIG. 1 und 2 zuvor beschriebenen chemischen Reaktors. Hierbei ist in dem Spalt, der sich zwischen dem Träger 30 und der Reaktorwand 10 befindet, zusätzliche Drahtgestrick-Maschenware 60 U-förmig eingelegt. In der hierdurch gebildeten Vertiefung ist weitere, elastisch vorgespannte Drahtgestrick-Maschenware 90, beispielsweise eine metallische Dichtschnur aus Maschenware, eingelegt. Durch die Vorspannung wird die Drahtgestrick-Maschenware 60 fixiert.

Weiterhin befindet sich zwischen der Schicht aus Katalysatorpartikeln 80 und der Drahtgestrick-Maschenware 50 eine Schicht aus Inertpartikeln 100.

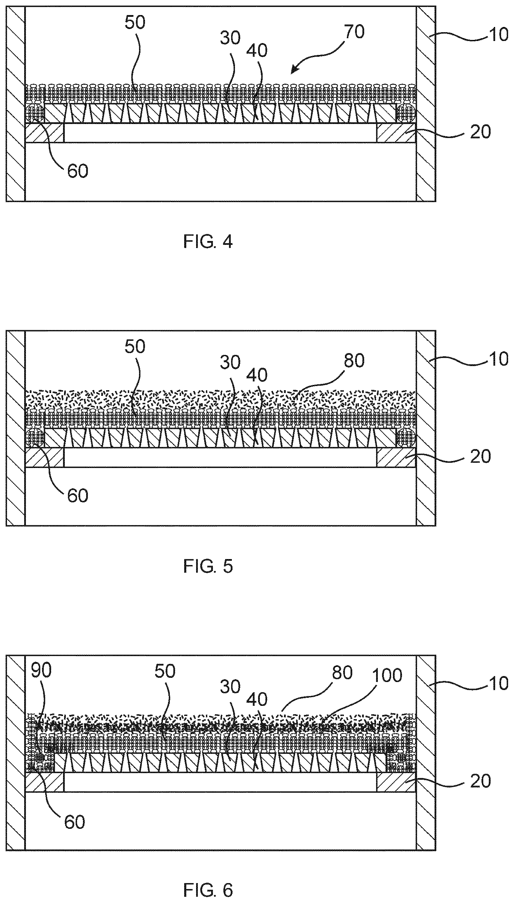

FIG. 4 zeigt eine zu dem in FIG. 1 gezeigten Reaktor analoge Einrichtung, bei der die auf dem Träger 30 liegende Drahtgestrick-Maschenware 50 jeweils bis zur Reaktorwand 10 durchgehend ausgebildet ist. Zusätzliche, rund ausgebildete Drahtgestrick-Maschenware 60 ist hierunter angeordnet und dichtet den Spalt zwischen Träger 30 und Reaktorwand 10 ebenfalls ab. Diese Ausgestaltung hat den Vorteil, dass die oben liegende Maschenwaren-Lage 50 zu Wartungs- und Inspektionszwecken leichter angehoben werden kann.

Die Anordnung in FIG. 5 entspricht derjenigen in FIG. 5, mit dem Unterschied, dass analog zu FIG. 2 nunmehr eine Schicht von Katalysatorpartikeln 80 auf der Drahtgestrick-Maschenware 50 platziert wurde.

FIG. 6 zeigt einen zu FIG. 3 analogen Reaktor, wobei auf einer Schicht von Inertpartikeln 100 die Katalysatorpartikel 80 angeordnet sind. Der Zwischenraum zwischen dem Träger 30 und der Reaktorwand 10 wird durch die zusätzliche Maschenware 60 abgedichtet. Diese Maschenware 60 ist U-förmig eingelegt, wobei die der Reaktorwand 10 zugewandte Seite des "U" senkrecht steht und die andere Seite des "U" dem Verlauf der Form des Trägers 30 folgt, also zunächst senkrecht steht und dann waagerecht weiter auf dem Träger 3 verläuft. Hierauf wird dann die Drahtgestrick-Maschenware 50 gelegt.

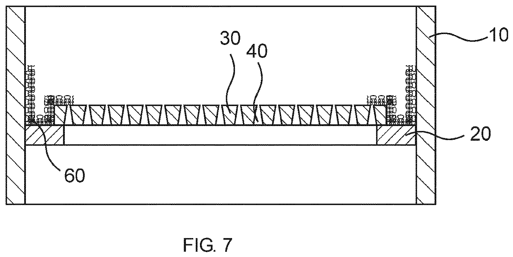

Zur besseren Übersicht ist dieser Verlauf in FIG. 7 dargestellt. Diese Figur entspricht FIG. 6, wobei nur die Reaktorwände 10, der Tragring 20, Träger 30, Spalten 40 und die zusätzliche Maschenware 60 gezeigt sind. In der durch das "U" der zusätzlichen Maschenware 60 gebildeten Vertiefung kann wieder, wie in FIG. 6 zu sehen, eine elastisch vorgespannte Drahtgestrick-Maschenware 90 eingelegt werden.

### Beispiele

Als Versuchsanlage für die Beispielreaktionen (Hydrierung von Nitrobenzol zu Anilin unter adiabaten Reaktionsbedingungen) diente ein Reaktor aus Edelstahl, der über einen Verdampfer mit Edukten beschickt wurde. Mittels Dosierpumpen wurde Nitrobenzol von oben in den Verdampfer gepumpt. Der Wasserstoff wurde von unten in den auf ca. 250 °C beheizten Verdampfer geleitet, so dass das von oben eingepumpte Nitrobenzol im Gegenstrom verdampfen konnte. Die Wasserstoff-Versorgung wurde vor dem Verdampfer durch Massedurchflussregler geregelt. Die Versuchsanlage wurde bei 4 bar betrieben und das Reaktionsgas vor Eintritt in den Reaktor in einem Wärmetauscher auf 240 °C temperiert.

Innerhalb des Reaktionsrohrs wurde eine 400 mm hohe Schüttung des Katalysators platziert. Einzelheiten zum jeweiligen Aufbau der Tragkonstruktion, die vor jedem Versuch erneuert wurde, finden sich in den entsprechenden Beispiel-Beschreibungen. Nach Austritt aus dem Reaktor wurde das Reaktionsprodukt mit Wasser gekühlt. Die nicht flüchtigen Bestandteile wurden so auskondensiert und in einem nachgeschalteten Abscheider von den gasförmigen Komponenten getrennt. Die flüssigen Bestandteile wurden aus dem Abscheider in einen Produktsammelbehälter geführt und dort aufgefangen. Vor dem Sammelbehälter befand sich eine Probennahmestelle, an der in regelmäßigen Zeitabständen Proben des Produkts gezogen werden konnten. Diese wurden gaschromatographisch auf die Anwesenheit von Nitrobenzol hin untersucht. Überschritt der Nitrobenzolgehalt im Rohprodukt 500 ppm, wurde die Reaktion beendet und der Katalysator regeneriert.

Die Belastung wurde in allen Beispielversuchen auf 1 g_{Nitrobenzol}/(ml_{Katalysator} · h) und das Wasserstoff: Nitrobenzol-Verhältnis auf ca. 80 : 1 festgelegt. Der Katalysator war ein Aluminiumoxid-geträgerter Metallkatalysator enthaltend 9 g/ l_{Träger} Pd, 9 g/ l_{Träger} V, 3 g/ l_{Träger} Pb auf α-Aluminiumoxid (siehe EP 0 011 090 A1). Die mittlere Partikelgröße x_{50,3} von Katalysatorpartikeln und erforderlichenfalls Inertpartikeln wurde mittels Siebanalyse bestimmt. Dabei kamen Siebe mit nominalen Sieböffnungsweiten von 1,25 mm, 1,4 mm, 1,6 mm, 1,7 mm und 1,8 mm ("kleine Kugeln", Katalysatorpartikel, Partikelgröße zwischen 1 mm und 2 mm) bzw. von 2,5 mm, 3,0 mm, 3,5 mm, 4,0 mm und 4,5 mm ("große Kugeln", Inertmaterial, Partikelgrößen zwischen 3,0 mm und 5,0 mm) zum Einsatz. Die jeweiligen x_{50,3} -Werte für Katalysator- und Inertpartikel wurden zu 1,5 mm bzw. 4 mm bestimmt.

Zur Durchführung der Reaktion wurde der Reaktor jeweils mit Stickstoff inertisiert und über einen Zeitraum von 48 h bei 240 °C mit Wasserstoff beaufschlagt. Dann wurde die Nitrobenzolzufuhr gestartet und über 5 Stunden auf den oben genannten Wert erhöht.

Zur Durchführung der Regeneration wurde der Reaktor wiederum mit Stickstoff inertisiert, auf 270 °C erwärmt und dann mit Luftstrom beaufschlagt, um Verkokungen abzubrennen. Dies wurde so lange durchgeführt, bis keine Wärmeabgabe mehr zu erkennen war und der CO₂-Gehalt im Abgasstrom auf weniger als 0,2 % gefallen war (bestimmt durch IR-Photometrie).

Zunächst wurden in jedem Beispiel acht Zyklen bestehend aus Reaktion und Regeneration gefahren, so dass der Reaktor und sein Inhalt erst von Umgebungstemperatur aufgeheizt wurden und dann mehreren Temperaturschwankungen zwischen 200 und 500°C ausgesetzt waren. Beim darauffolgenden Zyklus (im Folgenden Testzyklus genannt) wurde der Zeitpunkt des Nitrobenzoldurchbruchs festgehalten und der Reaktor nach der Regeneration auf Umgebungstemperatur abgekühlt und zur Inspektion geöffnet.

### Vergleichsbeispiel V-1: (Euroslot-Spaltsieb, Katalysator)

In diesem Beispiel wurde eine adiabate Hydrierung von Nitrobenzol zu Anilin in einem Reaktor durchgeführt, der mit einem selbsttragenden Spaltsieb für das Katalysatorbett ausgerüstet war. Der V-Profildraht des Spaltsiebes hatte eine Breite von 1,8 mm und eine Höhe von 3,7 mm und die Spaltweite zwischen den V-Profildrähten betrug 0,65 mm. Die Konstruktion der V-Profildrähte war normal, also nicht invers.

Das Spaltsieb war aus parallel angeordneten Segmenten ausgeführt. Jedes Segment lag auf einer Trägerstruktur und war mit dem nächsten Träger verschraubt. Die Verbindungen ragten über die Siebfläche hinaus. Korrosion sowie das Durchbiegen der Profile und Stützprofile wurden durch Verwendung von austenitischem Stahl verhindert. Der äußere Tragring des Siebs war schwimmend auf einem an der Reaktorwand befindlichen Auflagering gelagert. Dabei bestand im kalten Zustand ein Spalt von ca. 1 cm zwischen Tragring und Reaktorwand. Das Katalysatorfestbett, dessen Katalysatorpartikel eine mittlere Partikelgröße x_{50,3} von 1,5 mm hatten, kam auf dem Spaltsieb zu liegen.

Im Testzyklus wurde bereits nach 4 Tagen Reaktionszeit Nitrobenzol mit einer Konzentration von mehr als 500 ppm im Reaktionsprodukt gefunden. Bei der Inspektion des Reaktors wurden tiefe Thromben im Katalysatorbett festgestellt. Unterhalb der Thromben hatten sich V-Profildrähte des Spaltsiebes abgelöst. Entlang der Reaktorwand zeigte das Katalysatorbett ebenfalls eine Vertiefung.

Die fehlende Katalysatormenge aus dem Katalysatorfestbett wurde unterhalb des Spaltsiebes auf dem Reaktorboden und in den nachfolgenden Apparaten der Anlage gefunden.

### Vergleichsbeispiel V-2: (Euroslot-Spaltsieb, Inertmaterial, Katalysator)

Die Versuchsdurchführung entsprach der aus Beispiel V-1, wobei zusätzlich das Stützsieb mit zwei 5 cm hohen Schichten aus Inertmaterial belegt war. Die erste, auf dem Sieb liegende Schicht bestand aus Aluminiumoxid-Kugeln mit 6 mm Durchmesser, die darauf liegende Schicht aus Aluminiumoxid-Kugeln mit 4 mm Durchmesser. Erst auf dieser zweiten Schicht befand sich die Katalysatorschüttung, deren Katalysatorpartikel eine mittlere Partikelgröße x_{50,3} von 1,5 mm hatten.

Im Testzyklus wurde nach ca. 36 Tagen Reaktionszeit Nitrobenzol mit einer Konzentration von mehr als 500 ppm im Reaktionsprodukt gefunden. Bei der Inspektion des Reaktors wurden keine Thromben im Katalysatorbett festgestellt, obwohl wieder Schäden am Stützsieb beobachtet wurden. Entlang der Reaktorwand zeigte das Katalysatorbett keine Vertiefung. Am Reaktorboden und in den nachfolgenden Apparaten wurde weder Katalysator, noch Inertmaterial gefunden.

### Erfindungsgemäßes Beispiel E-1: (Spaltsieb, Drahtgestrick, Inertmaterial, Katalysator)

Die Versuchsdurchführung entsprach dem Vergleichsbeispiel V-1, wobei zusätzlich das Spaltsieb mit einer Drahtgestrick-Maschenware aus Endlosdraht belegt war. Die Maschenware hatte 0,23 mm Drahtdurchmesser, war zweifach gestrickt und dreilagig gefaltet. Die Maschenware war aus Edelstahl hergestellt, und die durchschnittliche lichte Maschenweite betrug 0,65 mm. Die Maschenware wurde mittels Bolzen an dem Spaltsieb befestigt.

Die Maschenware war mit einer 5 cm hohen Schicht von Inertmaterial aus Aluminiumoxidkugeln, die eine mittlere Partikelgröße x_{50,3} von 4 mm hatten, belegt.

Das Katalysatorfestbett, dessen Katalysatorpartikel eine mittlere Partikelgröße x_{50,3} von 1,5 mm hatten, kam auf dem Inertmaterial zu liegen.

Im Testzyklus wurde nach ca. 38 Tagen Reaktionszeit Nitrobenzol mit einer Konzentration von mehr als 500 ppm im Reaktionsprodukt gefunden. Bei der Inspektion des Reaktors wurden keine Thromben im Katalysatorbett festgestellt, obwohl wieder Schäden am Stützsieb beobachtet wurden. Entlang der Reaktorwand zeigte das Katalysatorbett keine Vertiefung. Am Reaktorboden und in den nachfolgenden Apparaten wurde weder Katalysator, noch Inertmaterial gefunden.

### Erfindungsgemäßes Beispiel E-2: (Spaltsieb, Drahtgestrick, Inertmaterial, met. Dichtschnur, Kat.)

Die Versuchsdurchführung entsprach dem Beispiel E-1, wobei zusätzlich in dem Spalt zwischen dem äußeren Tragring des Spaltsiebes und der Reaktorwand eine metallische Dichtschnur aus Drahtgestrick-Maschenware eingelegt war, deren Drahtdurchmesser 0,23 mm und deren durchschnittliche lichte Maschenweite 0,65 mm betrug. Die Dichtschnur war auch unter den Reaktionsbedingungen noch flexibel und hatte einen Durchmesser von 1,2 cm, so dass sie durch die eigene Federspannung im Spalt befestigt war.

Im Testzyklus wurde nach ca. 38 Tagen Reaktionszeit Nitrobenzol mit einer Konzentration von mehr als 500 ppm im Reaktionsprodukt gefunden. Bei der Inspektion des Reaktors wurden keine Thromben im Katalysatorbett festgestellt, obwohl wieder Schäden am Stützsieb beobachtet wurden. Entlang der Reaktorwand zeigte das Katalysatorbett keine Vertiefung. Am Reaktorboden und in den nachfolgenden Apparaten wurde weder Katalysator, noch Inertmaterial gefunden.

### Erfindungsgemäßes Beispiel E-3: (einfacher Rost, Drahtgestrick, metallische Dichtschnur, Katalysator)

In diesem Beispiel wurde eine adiabate Hydrierung von Nitrobenzol zu Anilin in einem Reaktor durchgeführt, der mit einem einfachen Rost für das Katalysatorbett ausgerüstet war. Die durchschnittliche Maschenweite des Rosts betrug 30 x 10 mm. Korrosion sowie das Durchbiegen der Profile und Stützprofile wurden durch Verwendung von austenitischem Stahl verhindert. Der äußere Tragring des Rosts war schwimmend auf einem an der Reaktorwand befindlichen Auflagering gelagert. Dabei war im kalten Zustand ein Spalt von ca. 1 cm zwischen Tragring und Reaktorwand. In diesen Spalt wurde eine metallische Dichtschnur aus Drahtgestrick-Maschenware eingelegt, deren Drahtdurchmesser 0,23 mm und deren durchschnittliche lichte Maschenweite 0,65 mm betrug. Die Dichtschnur war auch unter den Reaktionsbedingungen noch flexibel und hatte einen Durchmesser von 1,2 cm, so dass sie durch die eigene Federspannung im Spalt befestigt war.

Der Rost war mit einer Drahtgestrick-Maschenware aus Endlosdraht belegt. Die Maschenware hatte 0,23 mm Drahtdurchmesser, war zweifach gestrickt und dreilagig gefaltet. Die Maschenware war aus Edelstahl hergestellt, und die durchschnittliche lichte Maschenweite betrug 0,65 mm. Die Maschenware war mittels Bolzen an dem Rost befestigt.

Die Maschenwaren waren mit einer 5 cm hohen Schicht von Inertmaterial aus Aluminiumoxidkugeln, die eine mittlere Partikelgröße x_{50,3} von 4 mm hatten, belegt.

Das Katalysatorfestbett, dessen Katalysatorpartikel eine mittlere Partikelgröße x_{50,3} von 1,5 mm hatten, kam auf dem Inertmaterial zu liegen.

Im Testzyklus wurde nach ca. 900 h Reaktionszeit Nitrobenzol mit einer Konzentration von mehr als 500 ppm im Reaktionsprodukt gefunden. Bei der Inspektion des Reaktors wurden keine Thromben im Katalysatorbett festgestellt, die durchschnittliche lichte Maschenweite des Tragrosts war größer als der Durchmesser der Katalysator- bzw. Inertmaterialpartikel. Entlang der Reaktorwand zeigte das Katalysatorbett keine Vertiefung. Am Reaktorboden und in den nachfolgenden Apparaten wurde weder Katalysator, noch Inertmaterial gefunden.

### Erfindungsgemäßes Beispiel E-4: (wie Beispiel E-3, aber mit erhöhter Katalysatorschüttung)

Die Versuchsdurchführung entsprach dem Beispiel E-3 mit dem Unterschied, dass die Höhe der Katalysatorschüttung auf 50 cm erhöht wurde. Es wurde je Zeiteinheit die gleiche Menge Nitrobenzol umgesetzt wie in den Vergleichsbeispielen sowie den Beispielen E-1 bis E-3. Dadurch ergab sich für diesen Fall eine Volumenbelastung des Katalysators von nur 0,8 g_{Nitrobenzol}/(ml_{Katalysator} · h) statt 1 g _{Nitrobenzol}/(ml_{Katalysator} · h).

Im Testzyklus wurde nach ca. 46 Tagen Reaktionszeit Nitrobenzol mit einer Konzentration von mehr als 500 ppm im Reaktionsprodukt gefunden. Bei der Inspektion des Reaktors wurden keine Thromben im Katalysatorbett festgestellt, die durchschnittliche lichte Maschenweite des Tragrosts war größer als der Durchmesser der Katalysator- bzw. Inertmaterialpartikel. Entlang der Reaktorwand zeigte das Katalysatorbett keine Vertiefung. Am Reaktorboden und in den nachfolgenden Apparaten wurde weder Katalysator noch Inertmaterial gefunden.

Die Ergebnisse sind zur besseren Übersicht in der nachfolgenden Tabelle zusammengefasst:

| Beispiel | V-1 | V-2 | E-1 | E-2 | E-3 | E-4 |
|---|---|---|---|---|---|---|
| Spaltsieb (S) oder Rost (R) | S | S | S | S | R | R |
| Inertmaterial | | X | X | X | | |
| Gestrickauflage | | | X | X | X | X |
| Dichtschur am Reaktorrand | | | | X | X | X |
| Nitrobenzol-Durchschlag nach | 4 d | 36 d | 36 d | 38 d | 38 d | 46 d |
| Aufwand für Befüllen des Reaktors | gering | hoch | mittel | mittel | gering | gering |
| Aufwand für Fertigung der Tragkonstruktion | hoch | hoch | hoch | hoch | gering | gering |
| Aufwand für Instandhaltung der Tragkonstruktion | hoch | mittel | gering | gering | gering | gering |

## Patentansprüche

1. Chemischer Reaktor zur heterogen katalysierten Umsetzung eines Fluids, umfassend eine vom Fluid durchströmte Halteeinrichtung für Partikel (80, 100), **dadurch gekennzeichnet,**
**dass** die in Strömungsrichtung des Fluids gesehen stromaufwärts gelegene Seite der Halteeinrichtung Drahtgestrick-Maschenware (50) umfasst, auf der Katalysatorpartikel (80) angeordnet sind oder auf der eine Schicht von gegenüber dem Fluid inerten Partikeln (100) angeordnet ist und auf dieser Schicht Katalysatorpartikel (80) angeordnet sind, wobei die Drahtgestrick-Maschenware (50) auf einem Träger (30) angeordnet ist und der Träger (30) in Strömungsrichtung des Fluids durchgehende Öffnungen (40) umfasst,
und wobei die durchschnittliche lichte Maschenweite des Drahtgestricks (50) kleiner als die mittlere Partikelgröße x_{50.3} der Partikel (80, 100) ist.

2. Reaktor gemäß Anspruch 1, wobei die durchschnittliche lichte Maschenweite des Drahtgestricks (50) ≥ 20% bis ≤ 80% der mittleren Partikelgröße x_{50.3} der Partikel (80, 100) beträgt.

3. Reaktor gemäß Anspruch 1, wobei die Dehnbarkeit der Drahtgestrick-Maschenware (50) unter mechanischer und/oder thermischer Belastung in Flächenrichtung ≥ 1 % beträgt.

4. Reaktor gemäß Anspruch 1, wobei der Drahtdurchmesser der Drahtgestrick-Maschenware (50) ≥ 0,03 mm bis ≤ 1 mm beträgt.

5. Reaktor gemäß Anspruch 1, wobei die Höhe der Drahtgestrick-Maschenware (50) in der Halteeinrichtung für Partikel ≥ 3 mm bis ≤ 100 mm beträgt.

6. Reaktor gemäß Anspruch 1, wobei die Drahtgestrick-Maschenware (50) eine durch mechanische Einwirkung verformte Drahtgestrick-Maschenware ist.

7. Reaktor gemäß Anspruch 1, wobei zwischen dem Träger (30) und der Wand (10) des Reaktors zusätzlich Drahtgestrick-Maschenware (60) angeordnet ist.

8. Verfahren zur Umsetzung eines Fluids, **dadurch gekennzeichnet, dass** die Umsetzung in einem Reaktor gemäß einem der Ansprüche 1 bis 7 in Gegenwart von heterogenen Katalysatorpartikeln (80) durchgeführt wird und die Katalysatorpartikel (80) in der Halteeinrichtung für Katalysatorpartikel (80) angeordnet sind.

9. Verfahren gemäß Anspruch 8, wobei weiterhin nach Beendigung der Umsetzung der Reaktor in Gegenwart von Sauerstoff auf eine Temperatur von ≥ 200 °C bis ≤ 400 °C erhitzt wird.

10. Verfahren gemäß Anspruch 8, wobei das Fluid aromatische Nitroverbindungen und Wasserstoff umfasst.

11. Verfahren gemäß Anspruch 8, wobei die Umsetzung adiabatisch durchgeführt wird.

12. Verwendung von Drahtgestrick-Maschenware (50) in einer Halteeinrichtung für Katalysatorpartikel (80) oder für eine Schicht inerter Partikel (100), auf der Katalysatorpartikel (80) angeordnet sind, in chemischen Reaktoren, wobei die Halteeinrichtung einen Träger (30) aufweist, auf dem die Drahtgestrick-Maschenware (50) angeordnet ist.

13. Verwendung gemäß Anspruch 12, wobei der Träger ein Spaltsieb oder ein Gitterrost ist.

## Claims

1. Chemical reactor for the heterogeneously catalysed reaction of a fluid, comprising a retaining means, through which fluid flows, for particles (80, 100), **characterized in that** the side of the retaining means arranged upstream in terms of the direction of flow of the fluid comprises wire mesh knitted fabric (50) on which catalyst particles (80) are arranged or on which a layer of particles (100) inert to the fluid is arranged, and catalyst particles (80) are arranged on this layer, wherein the wire mesh knitted fabric (50) is arranged on a support (30) and the support (30) comprises continuous openings (40) in the direction of flow of the fluid, and wherein the average clear mesh size of the wire mesh (50) is less than the mean particle size x_{50.3} of the particles (80, 100).

2. Reactor according to Claim 1, wherein the average clear mesh size of the wire mesh (50) is ≥ 20% to ≤ 80% of the mean particle size x_{50.3} of the particles (80, 100) .

3. Reactor according to Claim 1, wherein the extensibility of the wire mesh knitted fabric (50) under mechanical and/or thermal load parallel to the surface is ≥ 1%.

4. Reactor according to Claim 1, wherein the wire diameter of the wire mesh knitted fabric (50) is ≥ 0.03 mm to ≤ 1 mm.

5. Reactor according to Claim 1, wherein the height of the wire mesh knitted fabric (50) in the particle retaining means is ≥ 3 mm to ≤ 100 mm.

6. Reactor according to Claim 1, wherein the wire mesh knitted fabric (50) is a wire mesh knitted fabric deformed by mechanical action.

7. Reactor according to Claim 1, wherein wire mesh knitted fabric (60) is additionally arranged between the support (30) and the wall (10) of the reactor.

8. Method for reacting a fluid, **characterized in that** the reaction is carried out in a reactor according to any one of Claims 1 to 7 in the presence of heterogeneous catalyst particles (80), and the catalyst particles (80) are arranged in the retaining means for catalyst particles (80).

9. Method according to Claim 8, wherein the reactor is heated, once the reaction has finished and in the presence of oxygen, to a temperature of ≥ 200°C to ≤ 400°C.

10. Method according to Claim 8, wherein the fluid comprises aromatic nitro compounds and hydrogen.

11. Method according to Claim 8, wherein the reaction is carried out adiabatically.

12. Use of wire mesh knitted fabric (50) in a retaining means for catalyst particles (80) or for a layer of inert particles (100) on which catalyst particles (80) are arranged in chemical reactors, wherein the retaining means has a support (30) on which the wire mesh knitted fabric (50) is arranged.

13. Use according to Claim 12, wherein the support is a wedge wire screen or a grate.

## Revendications

1. Réacteur chimique pour la mise en réaction sous catalyse hétérogène d'un fluide, comprenant un dispositif de maintien traversé par le fluide pour des particules (80, 100), **caractérisé en ce que**
le côté en amont dans la direction de l'écoulement du fluide du dispositif de maintien comprend un grillage métallique (50) sur lequel des particules de catalyseur (80) sont agencées ou sur lequel une couche de particules (100) inertes vis-à-vis du fluide est agencée et des particules de catalyseur (80) sont agencées sur cette couche, le grillage métallique (50) étant agencé sur un support (30) et le support (30) comprenant des ouvertures continues (40) dans la direction de l'écoulement du fluide,
et la largeur de maille libre moyenne du grillage métallique (50) étant inférieure à la taille de particule moyenne x_{50,3} des particules (80, 100).

2. Réacteur selon la revendication 1, dans lequel la largeur de maille libre moyenne du grillage métallique (50) est de ≥ 20 % à ≤ 80 % de la taille de particule moyenne x_{50,3} des particules (80, 100).

3. Réacteur selon la revendication 1, dans lequel la ductilité du grillage métallique (50) sous une charge mécanique et/ou thermique dans la direction de la surface est ≥ 1 %.

4. Réacteur selon la revendication 1, dans lequel le diamètre des fils du grillage métallique (50) est de ≥ 0,03 mm à ≤ 1 mm.

5. Réacteur selon la revendication 1, dans lequel la hauteur du grillage métallique (50) dans le dispositif de maintien pour particules est de ≥ 3 mm à ≤ 100 mm.

6. Réacteur selon la revendication 1, dans lequel le grillage métallique (50) est un grillage métallique déformé par action mécanique.

7. Réacteur selon la revendication 1, dans lequel un grillage métallique (60) est en outre agencé entre le support (30) et la paroi (10) du réacteur.

8. Procédé de mise en réaction d'un fluide, **caractérisé en ce que** la mise en réaction est réalisée dans un réacteur selon l'une quelconque des revendications 1 à 7 en présence de particules de catalyseur hétérogènes (80) et les particules de catalyseur (80) sont agencées dans le dispositif de maintien pour particules de catalyseur (80).

9. Procédé selon la revendication 8, dans lequel en outre, après la fin de la réaction, le réacteur est porté à une température de ≥ 200 °C à ≤ 400 °C en présence d'oxygène.

10. Procédé selon la revendication 8, dans lequel le fluide comprend des composés nitro aromatiques et de l'hydrogène.

11. Procédé selon la revendication 8, dans lequel la réaction est réalisée sous forme adiabatique.

12. Utilisation d'un grillage métallique (50) dans un dispositif de maintien pour particules de catalyseur (80) ou pour une couche de particules inertes (100) sur laquelle des particules de catalyseur (80) sont agencées, dans des réacteurs chimiques, le dispositif de maintien comprenant un support (30) sur lequel le grillage métallique (50) est agencé.

13. Utilisation selon la revendication 12, dans laquelle le support est un tamis à fentes ou une grille.
